# FASCICULE DE BREVET EUROPEEN

(11) **EP 1 366 741 B1**
(45) Date de publication et mention de la délivrance du brevet: **17.11.2004**
(21) Numéro de dépôt: 03010664.5
(22) Date de dépôt: 13.05.2003
(51) Int. Cl.: A61K 7/06

(54) **Shampooings contenant au moins un copolymère séquencé amphiphile et au moins un polymère cationique ou amphotère**
Haarwaschmittel enthaltend mindestens ein amphiphiles folgerichtiges Copolymer und mindestens ein kationisches oder amphoteres Polymer
Shampoos containing at least one amphiphilic sequenced copolymer and at least one cationic or amphoteric polymer

(30) Priorité: 31.05.2002 FR 0206731
(43) Date de publication de la demande: 03.12.2003
(73) Titulaire: L'OREAL, 75008 Paris (FR)
(72) Inventeur: Dubief, Claude, 78150 Le Chesnay (FR); Restle, Serge, 95390 Saint-Prix (FR); Giroud, Franck, 92110 Clichy (FR)
(74) Mandataire: Le Blainvaux Bellegarde, Françoise

(56) Documents cités:
- EP-A- 0 494 022
- EP-A- 1 092 420
- WO-A-01/96429
- FR-A- 2 709 954
- US-A- 3 907 984

## Description

La présente invention concerne des compositions moussantes et détergentes, destinées à laver, conditionner et coiffer les cheveux, contenant l'association d'au moins un copolymère séquencé amphiphile et d'au moins un polymère cationique ou amphotère.

Les polymères cationiques sont, de loin, les composés les plus utilisés dans des shampooings pour faciliter le démêlage des cheveux humides et améliorer leur douceur après séchage voir par exemple EP-A-1 092 420. Ces polymères présentent toutefois un pouvoir coiffant médiocre et ne permettent pas de donner du corps au cheveux.

L'association de tels polymères cationiques conditionnants à des polymères fixants anioniques aboutit, certes, à une amélioration des performances de coiffage des shampooings (corps et maintien) mais modifie défavorablement le toucher des cheveux en les rendant secs et rêches.

L'utilisation de silicones ou d'un mélange de silicones et de polymères cationiques dans des shampooings coiffants assure un bon démêlage des cheveux, mais leur confère un toucher excessivement soyeux, non recherché pour de tels shampooings coiffants.

Les copolymères séquencés à blocs de silicone, utilisés seuls ou associés à des polymères cationiques comme décrit par exemple dans FR-A-2 709 954, facilitent le démêlage et améliorent le pouvoir coiffant des shampooings, mais donnent également un toucher trop soyeux.

Les polyuréthanes à blocs polyester ou polyéther, éventuellement associés à des polymères cationiques filmogènes, induisent, quant à eux, un toucher cireux parfois collant.

Les copolymères séquencés d'oxyde d'éthylène et d'oxyde de propylène sont pratiquement sans action bénéfique sur les cheveux.

L'utilisation de copolymères séquencés ramifiés dans des shampooings est dècrite dans WO-A-0 196 429. L'utilisation de copolymères séquencés linéaire dans des compositions coiffantes est décrite dans US-A-3 907 984.

La demanderesse a découvert, avec surprise, que l'utilisation d'un polymère cationique en association avec un copolymère séquencé linéaire, amphiphile, dans une base particulière pour shampooings permet d'obtenir des produits qui, à la fois, améliorent le démêlage des cheveux à l'état humide, donnent du corps aux cheveux séchés, facilitent la mise en forme de la coiffure et le maintien de celle-ci, sans altérer l'aspect lisse et brillant et le toucher agréable des cheveux traités.

La présente invention a par conséquent pour objet une composition de lavage des matières kératiniques, en particulier des fibres kératiniques, contenant, dans un milieu aqueux ou hydroalcoolique cosmétiquement acceptable,
- au moins un copolymère linéaire séquencé comprenant au moins un bloc hydrophobe et au moins un bloc hydrophile, à l'exclusion des copolymères séquencés d'oxyde d'éthylène et d'oxyde de propylène, des copolymères séquencés à motifs uréthanne et des copolymères séquencés à motifs siloxane,
- au moins un polymère cationique ou amphotère, et
- au moins un agent tensioactif anionique associé à au moins un agent tensioactif non-ionique et/ou au moins un agent tensioactif amphotère.

L'invention a en outre pour objet l'utilisation d'une telle composition pour le lavage des matières kératiniques, en particulier des fibres kératiniques.

Les copolymères linéaires séquencés utilisables selon la présente invention sont des copolymères dits "amphiphiles", à savoir des copolymères comportant à la fois des blocs hydrophobes et des blocs hydrophiles.

On entend par blocs hydrophobes selon la présente invention des blocs comprenant au moins 75 % en moles de monomères insolubles dans l'eau et par blocs hydrophiles des blocs comprenant au moins 75 % en moles de monomères hydrosolubles.

Un monomère "hydrosoluble" au sens de la présente invention, est un monomère qui, lorsqu'on l'introduit dans de l'eau à une température de 25 °C et à une concentration en poids égale à 0,5 %, éventuellement neutralisé, permet l'obtention d'une solution macroscopiquement homogène et transparente, c'est-à-dire ayant une valeur de transmittance de la lumière, à une longueur d'onde égale à 500 nm, à travers un échantillon de 1 cm d'épaisseur, d'au moins 70 %, de préférence d'au moins 80 %.

Les monomères hydrosolubles formant le ou les blocs hydrophiles des copolymères séquencés utilisés dans la présente invention peuvent être de nature anionique, non-ionique ou cationique et peuvent être utilisés seuls ou sous forme de mélange contenant deux ou plusieurs monomères différents.

On peut citer à titre d'exemples de monomères hydrosolubles anioniques, les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropanesulfonique, l'acide styrènesulfonique, l'acide vinylsulfonique et l'acide vinylphosphonique.

Les monomères hydrosolubles non-ioniques englobent, entre autres, l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle.

Enfin, les monomères hydrosolubles cationiques englobent par exemple le chlorure de diméthyldiallylammonium, le chlorure de méthylvinylimidazolium, la 2-vinylpyridine, la 4-vinylpyridine, la 2-méthyl-5-vinylpyridine, les halogénures de N-(alkyle en C₁₋₄)-4-vinylpyridinium tels que l'iodure de N-méthyl-4-vinylpyridinium, la vinylamine, les monomères de formule

H₂C=CR₁-CO-X₂

dans laquelle
R₁ représente un atome d'hydrogène ou un groupe méthyle,
X₂ représente un groupe hydrocarboné linéaire ou ramifié en C₁₋₆ portant au moins une fonction amine primaire, secondaire ou tertiaire ou au moins un atome d'azote quaternaire, ou un groupe de formule NHR₂ ou de formule NR₂R₃ où R₂ et R₃ représentent indépendamment l'un de l'autre chacun un groupe hydrocarboné en C₁₋₆, linéaire ou ramifié, portant au moins une fonction amine primaire, secondaire ou tertiaire ou au moins un atome d'azote quaternaire.

Les monomères insolubles dans l'eau formant le ou les blocs hydrophobes des copolymères séquencés sont choisis de préférence parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C₁₋₁₀, d'aryle en C₆₋₁₀ ou d'aralkyle en C₇₋₂₀ et les méthacrylates d'alkyle en C₁₋₁₀, d'aryle en C₆₋₁₀ ou d'aralkyle en C₇₋₂₀, comme par exemple les (meth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroacrylates d'alkyle.

Comme indiqué ci-dessus à propos de la définition des blocs hydrophobes et hydrophiles des copolymères séquencés, les monomères insolubles dans l'eau et les monomères hydrosolubles représentent au moins 75 % en moles respectivement des blocs hydrophobes et hydrophiles. Autrement dit, chaque bloc hydrophobe peut comprendre jusqu'à 25 % en moles d'un ou de plusieurs monomères hydrosolubles. Cette proportion est de préférence au plus égale 10 % en moles et idéalement inférieure ou égale à 5 % en moles.

De manière analogue, chaque bloc hydrophile peut comprendre jusqu'à 25 % en moles, de préférence jusqu'à 10 % en moles, et idéalement jusqu'à 5 % en moles, d'un ou de plusieurs monomères insolubles dans l'eau.

Les copolymères linéaires séquencés utilisés englobent bien entendu également ceux dans lesquels les blocs hydrophiles et les blocs hydrophobes sont constitués exclusivement respectivement de monomères hydrosolubles et de monomères insolubles dans l'eau. Ces blocs peuvent être des blocs homopolymères ou des blocs copolymères renfermant deux ou plus de deux monomères différents du même type.

La masse moléculaire moyenne en nombre de chaque bloc, qu'il soit hydrophobe ou hydrophile, copolymère ou homopolymère, est de préférence comprise entre 500 et 100 000, en particulier entre 500 et 50 000, avec un indice de polydispersité (M_{w}/Mₙ) compris entre 1,01 et 3,0, de préférence entre 1,1 et 2,5.

Les copolymères linéaires séquencés amphiphiles utilisés dans la présente invention peuvent être
- des copolymères diblocs de formule AB,
- des copolymères triblocs de formule ABA ou BAB et
- des copolymères multiblocs comprenant, au moins deux blocs hydrophiles et au moins deux blocs hydrophobes disposés en alternance, chaque A représentant un bloc hydrophile et chaque B représentant un bloc hydrophobe, les blocs A d'un même polymère pouvant être identiques ou différents et les blocs B d'un même polymère pouvant être identiques ou différents.

On préfère en particulier des copolymères diblocs et des copolymères triblocs comportant un bloc central hydrophile et deux blocs latéraux hydrophobes.

Les shampooings de la présente invention contiennent de préférence les copolymères séquencés amphiphiles à l'état dissous ou finement dispersé, autrement dit ces polymères sont de préférence solubles ou finement dispersibles dans milieu cosmétiquement acceptable.

Par "soluble" ou "finement dispersible" dans un milieu donné, on entend dans la présente demande, des polymères qui, introduits dans un tel milieu à une température de 25°C, éventuellement neutralisés, et à une concentration en poids égale à 0,1%, permettent l'obtention d'une solution ou suspension macroscopiquement homogène et transparente ou translucide, c'est-à-dire ayant une valeur de transmittance de la lumière, à une longueur d'onde égale à 500 nm, à travers un échantillon de 1 cm d'épaisseur, d'au moins 70%, de préférence d'au moins 80%.

Les copolymères linéaires séquencés sont de préférence hydrosolubles, éventuellement sous forme neutralisée.

La concentration des copolymères linéaires séquencés dans les shampooings de la présente invention est de préférence comprise entre 0,01 et 20 %, en particulier entre 0,1 à 5 %, rapportée au poids total de la composition de lavage.

Les polymères blocs de l'invention peuvent être préparés par les procédés de synthèse classiquement utilisés pour obtenir des polymères blocs. On peut citer par exemple les polymérisations anionique ou cationique, et la polymérisation radicalaire contrôlée (voir "*New Method ofPolymer Synthesis*", Blackie Academic & Professional, Londres, 1995, volume 2, page 1, ou Trends Polym. Sci. 4, page 183 (1996) de C. J. Hawker), qui peut être mise en oeuvre suivant différents procédés comme par exemple la polymérisation radicalaire par transfert d'atomes (*Atom Transfert Radical Polymerization* ou ATRP) (voir *JACS,* 117, page 5614 (1995), de Matyjasezwski *et al.*)*,* la méthode des radicaux tels que les nitroxydes (Georges et al., Macromolecules, 1993, 26, 2987).

On peut aussi utiliser ces procédés pour obtenir un seul des deux types de blocs du polymère de l'invention, l'autre bloc étant introduit dans le polymère final par l'intermédiaire de l'amorceur utilisé ou bien par réaction de couplage entre les blocs hydrophiles et hydrophobes.

Les polymères cationiques ou amphotères utilisés, conformément à l'invention, en association avec les copolymères séquencés amphiphiles décrits ci-dessus peuvent être des polymères synthétiques ou des polymères obtenus par modification chimique de polysaccharides.

Les polymères cationiques sont choisis par exemple parmi ceux décrits dans les demandes de brevet EP 0 337 354, FR 2 270 846, FR 2 383 660, FR 2 598 611, FR 2 470 596 et FR 2 519 863.

Les polymères cationiques préférés sont choisis parmi ceux qui contiennent des motifs comportant des groupements amines primaires, secondaires, tertiaires et/ou quaternaires qui font partie de la chaîne macromoléculaire principale, ou bien sont portés par des groupes latéraux directement reliés à celle-ci.

Parmi les polymères cationiques, on peut citer plus particulièrement les polymères du type polyamine, polyaminoamide et polyammonium quaternaire. Il s'agit de produits connus.
Les polymères du type polyamine, polyaminoamide et polyammonium quaternaire, que l'on peut utiliser dans les compositions de la présente invention, sont ceux décrits dans les brevets FR 2 505 348 et FR 2 542 997.

Parmi les polymères cationiques synthétiques, on peut citer en particulier les familles suivantes :
(1) les homopolymères ou copolymères d'esters ou d'amides acryliques ou méthacryliques à fonctions amine, comportant des motifs de formules suivantes: 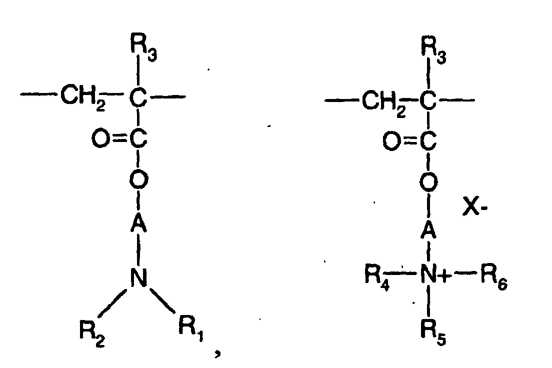 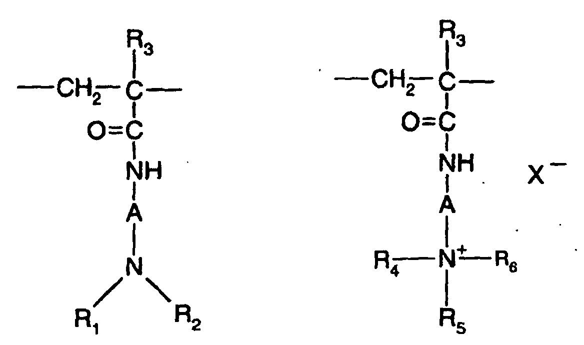 dans lesquelles:
   R₃, identiques ou différents, désignent un atome d'hydrogène ou un groupe CH₃ ;
   A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone; ,
   R₁ et R₂, identiques ou différents, représentent un atome d'hydrogène ou un groupe alkyle ayant de 1 à 6 atomes de carbone, et de préférence un groupe méthyle ou éthyle;
   X⁻ désigne un anion dérivé d'un acide minéral ou organique tel qu'un anion méthosulfate ou un halogénure comme le chlorure ou le bromure.

   Les copolymères de la famille (1) peuvent contenir en outre un ou plusieurs motifs dérivant de comonomères choisis dans la famille des acrylamides, méthacrylamides, diacétone-acrylamides, acrylamides et méthacrylamides substitués sur l'atome d'azote par des groupes alkyle inférieur en C₁₋₄, des groupes dérivés des acides acryliques ou méthacryliques ou de leurs esters, de vinyllactames tels que la vinylpyrrolidone ou le vinylcaprolactame, d'esters vinyliques.
   Parmi ces copolymères de la famille (1), on peut citer en particulier :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyl-triméthylammonium décrits, par exemple, dans la demande de brevet EP-A-080976 et vendus sous la dénomination BINAQUAT® P 100 par la société CIBA GEIGY,
   - le copolymère d'acrylamide et de méthosulfate de méthacryloyloxy-éthyltriméthylammonium vendu sous la dénomination RETEN® par la société HERCULES,
   - les copolymères vinylpyrrolidone/acrylate ou méthacrylate de dialkylaminoalkyle quaternisés ou non, tels que les produits vendus sous la dénomination GAFQUAT® par la société ISP comme, par exemple, GAFQUAT® 734 ou GAFQUAT® 755, ou bien les produits dénommés COPOLYMER 845, 958 et 937. Ces polymères sont décrits en détail dans les brevets FR 2 077 143 et FR 2 393 573,
   - les terpolymères méthacrylate de diméthylaminoéthyle/vinylcaprolactame/vinylpyrrolidone tels que le produit vendu sous la dénomination GAFFIX® VC 713 par la société ISP,
   - les copolymères vinylpyrrolidone/méthacrylamidopropyl-diméthylamine commercialisés notamment sous la dénomination STYLEZE® CC 10 par ISP, et
   - les copolymères vinylpyrrolidone/méthacrylamide de diméthylaminopropyle quaternisé, tels que le produit vendu sous la dénomination GAFQUAT® HS 100 par la société ISP.
(2) Les polymères formés de motifs pipérazinyle et de groupes alkylène ou hydroxyalkylène à chaîne droite ou ramifiée, éventuellement interrompue par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2 162 025 et FR 2 280 361.
(3) Les polyaminoamides solubles dans l'eau, préparés en particulier par polycondensation d'un composé acide avec une polyamine. Ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé, l'agent réticulant étant utilisé dans des proportions allant de 0,025 à 0,35 mole par groupement amine du polyaminoamide. Ces polyaminoamides peuvent être alkylés ou, s'ils comportent une ou plusieurs fonctions amines tertiaires, quaternisées. De tels polymères sont notamment décrits dans les brevets FR 2 252 840 et FR 2 368 508.
(4) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle ou propyle, et le groupe alkylène comporte de 1 à 4 atomes de carbone, et désigne de préférence le groupe éthylène. De tels polymères sont notamment décrits dans le brevet FR 1 583 363.
   Parmi ces dérivés, on peut citer plus particulièrement les polymères acide adipique/diméthylaminohydroxypropyl-diéthylène-triamine vendus sous la dénomination Cartaretine® F, F4 ou F8 par la société Sandoz.
(5) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone, le rapport molaire de la polyalkylène-polylamine à l'acide dicarboxylique étant compris entre 0,8:1 et 1,4:1. Le polyaminoamide résultant de cette réaction est ensuite amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets US 3 227 615 et US 2 961 347.
   Des polymères de ce type sont en particulier commercialisés sous la dénomination Hercosett® 57 par la société Hercules Inc. ou bien sous la dénomination de PD 170 ou Delsette® 101 par la société Hercules dans le cas du copolymère d'acide adipique/époxypropyl-diéthylène-triamine.
(6) Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 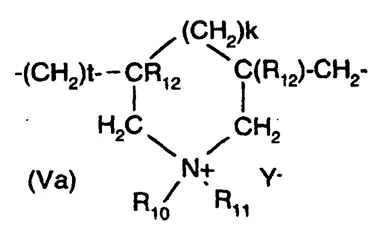 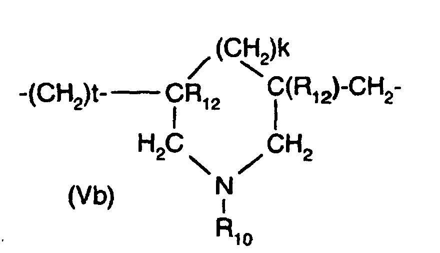 dans lesquelles
   k et t sont égaux à 0 ou 1, la somme k + t étant égale à 1 ;
   R₁₂ désigne un atome d'hydrogène ou un groupe méthyle ;
   R₁₀ et R₁₁, indépendamment l'un de l'autre, désignent un groupement alkyle ayant de 1 à 6 atomes de carbone, un groupement hydroxyalkyle en C₁₋₅, un groupement amidoalkyle inférieur en C₁-C₄, ou bien R₁₀ et R₁₁ peuvent désigner conjointement avec l'atome d'azote auquel ils sont rattachés, des groupements hétérocycliques, tels que pipéridinyle ou morpholinyle ;
   Y est un anion tel que bromure, chlorure, acétate, borate, citrate, tartrate, bisulfate, bisulfite, sulfate, phosphate.
   Ces polymères sont notamment décrits dans le brevet FR 2 080 759 et dans son certificat d'addition 2 190 406.
   Parmi les polymères définis ci-dessus, on peut citer plus particulièrement l'homopolymère de chlorure de diméthyldiallyl-ammonium vendu sous la dénomination MERQUAT® 100 par la société NALCO (et ses homologues de faibles masses moléculaires moyenne en poids) et les copolymères de chlorure de diallyldiméthylammonium et d'acrylamide commercialisés sous la dénomination MERQUAT® 550.
(7) Les polymères de diammonium quaternaire contenant des motifs récurrents répondant à la formule (VI) : 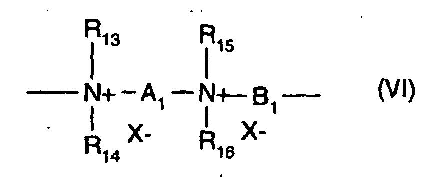 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkyle aliphatiques inférieurs, ou bien R₁₃, R₁₄, R₁₅ et R₁₆, ensemble ou séparément, forment avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁₋₆, linéaire ou ramifié, substitué par un groupement nitrile, ester, acyle, amide ou -CO-O-**R**₁₇-D ou -CO-NH-R₁₇-D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-D-OC-(CH₂)ₙ-

      dans lequel D désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH- .

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets FR 2 320 330, FR 2 270 846, FR 2 316 271, FR 2 336 434 et FR 2 413 907 et les brevets US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 et US 4 027 020.
   On peut utiliser plus particulièrement les polymères qui sont constitués de motifs récurrents répondant à la formule : 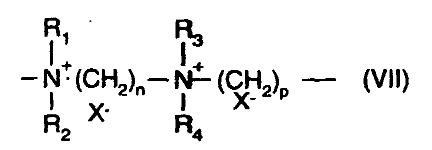 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (VII) particulièrement préféré est celui pour lequel R₁, R₂, R₃ et R₄ représentent un groupe méthyle et n=3, p=6 et X=Cl, dénommé chlorure d'hexadiméthrine (CTFA).
(8) Les polymères de polyammonium quaternaire, constitués de motifs de formule (VIII) : 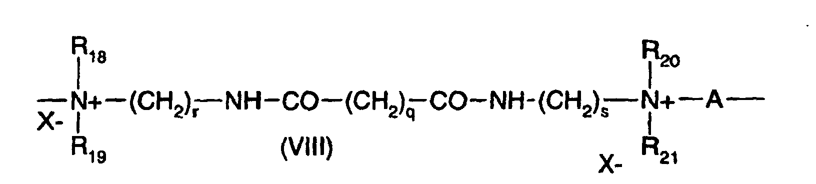 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut citer parmi ceux-ci, par exemple, les produits Mirapol® A 15, Mirapol® AD1, Mirapol® AZ 1 et Mirapol® 175 vendus par la société Miranol.
(9) Les polymères quaternaires de vinylpyrrolidone et de vinylimidazole tels que, par exemple, les produits commercialisés sous les dénominations Luviquat® FC 905, FC 550 et FC 370 par la société B.A.S.F. On peut citer notamment les copolymères de vinylpyrrolidone et de chlorure de méthylvinylimidazolium.
(10) Les polyamines comme le Polyquart® H vendu par HENKEL, référencées sous le nom de POLYETHYLENEGLYCOL (15) TALLOW POLYAMINE dans le dictionnaire CTFA.
(11) Les polymères réticulés ou non réticulés de sels de méthacryloyloxyalkyl(C₁₋₄) trialkyl(C₁₋₄)ammonium, tels que les polymères obtenus par homopolymérisation du méthacrylate de diméthylaminoéthyle quaternisé par le chlorure de méthyle, ou par copolymérisation de l'acrylamide et de méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide. On peut plus particulièrement utiliser un copolymère réticulé acrylamide/chlorure de méthacryloyloxy-éthyl-triméthylammonium (20/80 en poids) sous forme de dispersion contenant 50 % en poids dudit copolymère dans de l'huile minérale. Cette dispersion est commercialisée sous le nom de SALCARE® SC 92 par la Société CIBA. On peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont commercialisées sous les noms de SALCARE® SC 95 et SALCARE® SC 96 par la Société CIBA.

Les polymères cationiques polysaccharidiques englobent par exemple les familles suivantes :
(1) Les dérivés d'éthers de cellulose comportant des groupements ammonium quaternaires décrits dans le brevet FR 1 492 597, et en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammoniums quaternaires d'hydroxyéthylcellulose ayant réagi avec un époxyde substitué par un groupement triméthylammonium.
(2) Les dérivés cationiques de la cellulose tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl-triméthylammonium, de méthacrylamidopropyltriméthylammonium, de diméthyldiallylammonium.
   Les produits commercialisés répondant à cette définition sont plus particulièrement les produits vendus sous la dénomination Celquat® L 200 et Celquat® H 100 par la Société National Starch.
(3) Les polysaccharides cationiques décrits plus particulièrement dans les brevets US 3 589 578 et US 4 031 307, tels que les gommes de guar cationiques par exemple contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR® C 13 S, JAGUAR® C 15, JAGUAR® C 17 ou JAGUAR® C 162 par la société MEYHALL.
(4) les chitosanes et leurs sels tels que l'acétate, le lactate, le glutamate, le gluconate ou le pyrrolidone-carboxylate de chitosane.
   Parmi ces composés, on peut citer le chitosane ayant un taux de désacétylation de 90,5 % en poids commercialisé sous la dénomination KYTAN BRUT STANDARD par la société ABER TECHNOLOGIES, le pyrrolidone-carboxylate de chitosane vendu sous la dénomination KYTAMER® PC par la société AMERCHOL.

Les polymères amphotères synthétiques englobent notamment :
(1) les copolymères de chlorure de diméthyldiallylammonium et d'acide acrylique, commercialisés par exemple sous les dénominations Merquat® 280 et Merquat® 295 par la société NALCO ;
(2) les terpolymères de chlorure de diméthyldiallylammonium, d'acrylamide et d'acide acrylique, commercialisés par exemple sous la dénomination Merquat® Plus 3330 par la société NALCO ;
(3) les terpolymères de chlorure d'acrylamidopropyltriméthylammonium, d'acrylamide et d'acide 2-amidopropanesulfonique, commercialisés par exemple sous la dénomination Bozequat® 4000 par la société Hoechst, et
(4) les terpolymères de chlorure de méthacrylamidopropyltriméthylammonium, d'acrylate de méthyle et d'acide acrylique, commercialisés par exemple sous la dénomination Merquat® 2001 par la société NALCO.

Les polymères amphotères dérivés de polysaccharides englobent par exemple les familles suivantes :
(1) les gommes de guar portant à la fois des groupes cationiques tels que amine primaire, secondaire ou tertiaire, ammonium, sulfonium ou phosphonium, et des groupes anioniques tels que carboxyle, sulfonate, sulfate, phosphate ou phosphonate, préparées conformément au procédé décrit dans la demande de brevet EP 0 943 627 ;
(2) les dérivés amphotères d'éthers de cellulose, décrits dans la demande internationale WO 90/03779, comprenant en moyenne, par motif de glucose, au moins 0,1 groupe à fonction amine ou ammonium de formule (I) ci-dessous, et au moins 0,1 groupe à fonction carboxyle de formule (II) ci-dessous :

   (I) -[(CH₂)ₘ-N⁺R¹R²]ₓ-R³ (II) -CₙH₂ₙ-COO⁻

   dans lesquelles m = 2 - 4, n = 1 - 3, x = 0 - 3, R¹ et R² représentent chacun un groupe alkyle en C₁₋₄, R³ représente un groupe -(CH₂)ₘ-NR¹R² ou -(CH₂)ₘ-N⁺R¹R²R⁴ où R⁴ représente un groupe alkyle en C₁₋₄ ou un groupe -CₙH₂ₙ-COO⁻ ;
(3) les polymères dérivés du chitosane comportant des motifs répondant aux formules suivantes : 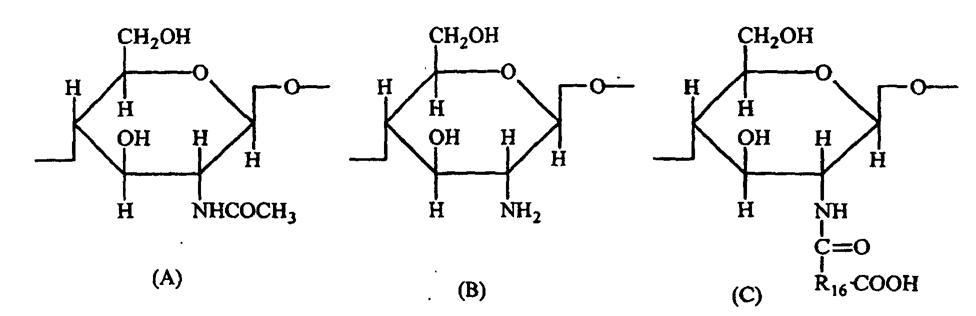 le motif (A) étant présent dans des proportions comprises entre 0 et 30%, le motif (B) dans des proportions comprises entre 5 et 50% et le motif (C) dans des proportions comprises entre 30 et 90%, étant entendu que dans ce motif (C), R₁₆ représente un groupe de formule : 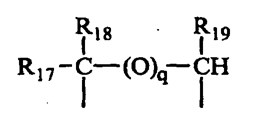 dans laquelle si q = 0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q = 1, R₁₇, R₁₈ et R₁₉ représentent chacun un atome d'hydrogène, ainsi que les sels formés par ces composés avec des bases ou des acides ;
(4) Les polymères obtenus par N-carboxylation du chitosane tels que le N-carboxyméthylchitosane ou le N-carboxybutylchitosane vendu sous la dénomination EVALSAN® par la société JAN DEKKER.

Les polymères cationiques ou amphotères de l'invention sont de préférence filmogènes.

Les polymères cationiques ou amphotères sont généralement présents dans les shampooings de la présente invention en une concentration allant de 0,001 % à 20 % en poids, de préférence de 0,01 à 5 % en poids, rapportée au poids total de la composition.

L'association des deux types de polymères essentiels pour la présente invention décrits ci-dessus (copolymère séquencé + polymère cationique ou amphotère), se trouve dans une base pour shampooings spécifique contenant l'association d'au moins un agent tensioactif anionique et d'au moins un agent tensioactif non-ionique et/ou d'au moins un agent tensioactif amphotère.

Les agents tensioactifs anioniques, non-ioniques et amphotères utilisables dans les compositions de lavage de la présente invention sont connus et couramment utilisés dans le domaine cosmétique.

Comme agents tensioactifs anioniques utilisables dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkylarylsulfonates, les α-oléfine-sulfonates, les paraffme-sulfonates, les alkylsulfosuccinates, les alkyléthersulfosuccinates, les alkylamidesulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides alkyl(C₆₋₂₄)éther-carboxyliques poly-oxyalkylénés, les acides alkyl(C₆₋₂₄)aryl(C₆₋₂₄)éther-carboxyliques poly-oxyalkylénés, les acides alkyl(C₆₋₂₄)amidoéther-carboxyliques poly-oxyalkylénés et leurs sels, en particulier ceux comportant de 2 à 50 motifs oxyde d'éthylène, et leurs mélanges.

On utilise de préférence les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

Les agents tensioactifs amphotères, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les alkyl(C₈₋₂₀)amidoalkyl(C₆₋₈)-bétaïnes ou les alkyl(C₈₋₂₀)amidoalkyl(C₆₋₈)sulfobétaïnes.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   Rₐ'-CONHCH₂CH₂-N(B)(C) (2)

   dans laquelle :
   B représente -CH₂CH₂OX',
   C représente -(CH₂)_{z}-Y', avec z = 1 ou 2,
   X' représente le groupe -CH₂CH₂-COOH ou un atome d'hydrogène,
   Y' représente -COOH ou le groupe -CH₂-CHOH-SO₃H,
   Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré.

Parmi les tensioactifs amphotères, on utilise de préférence les (alkyle en C₈₋₂₀)-bétaïnes, les (alkyle en C₈₋₂₀)-amido(alkyle en C₆₋₈)bétaïnes, les alkylamphodiacétates et leurs mélanges.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyle en C₆₋₂₄)polyglycosides, les dérivés de N-(alkyle en C₆₋₂₄)glucamine, les oxydes d'amines tels que les oxydes d'(alkyle en C₁₀₋₁₄)amines ou les oxydes de N-(acyle en C₁₀₋₁₄)-aminopropylmorpholine.

Parmi les tensioactifs non-ioniques cités ci-dessus, on utilise de préférence les (alkyle en C₆₋₂₄)polyglycosides.

La quantité d'agents tensioactifs anioniques est de préférence comprise entre 3 % et 35 % en poids, en particulier entre 5 % et 25 % en poids, rapportée au poids total de la composition cosmétique.

La quantité totale d'agents tensioactifs amphotères et/ou non ioniques, est de préférence comprise entre 0,5 et 30 %, et en particulier entre 1 et 20 % rapportée au poids total de la composition.

Le pH des compositions de lavage de la présente invention est de préférence compris entre 2 et 11, et en particulier entre 3 et 10.

Le milieu liquide des compositions de l'invention est aqueux ou hydroalcoolique, c'est-à-dire que, dans ce dernier cas, les compositions contiennent en plus d'une phase aqueuse, un ou plusieurs solvants choisis parmi les alcools inférieurs tels que l'éthanol ou l'isopropanol, et les polyols tels que le glycérol, le propylèneglycol et les polyéthylèneglycols.

Les compositions selon l'invention peuvent également contenir des principes actifs cosmétiques ou des additifs de formulation tels que des épaississants polymériques naturels ou synthétiques, anioniques, amphotères, zwittérioniques, non ioniques ou cationiques, associatifs ou non, des épaississants non polymériques comme des acides ou des électrolytes, des agents tensioactifs cationiques, des agents nacrants, des agents opacifiants, des colorants ou pigments, des parfums, des huiles minérales, végétales et/ou synthétiques, des silicones solubles, dispersibles ou insolubles, des cires dont les céramides, des vitamines, des filtres UV, des agents anti-radicalaires, des agents plastifiants, des agents conservateurs ou des agents de stabilisation du pH.

L'homme de métier veillera à choisir les éventuels additifs et leur quantité de manière à ce qu'ils ne nuisent pas aux propriétés intéressantes des compositions de lavage des fibres kératiniques de la présente invention.

Les compositions de l'invention peuvent éventuellement se présenter sous forme aérosol.

L'invention est illustrée à l'aide des exemples suivants.

### Exemple 1

On a préparé les deux shampooings A et B suivants :

| | Shampooing A | Shampooing B |
|---|---|---|
| Lauryléthersulfate de sodium (2OE) | 17 % m.a. | 17 % m.a. |
| Cocobétaïne | 2,5 % m.a. | 2,5 % m.a. |
| JR 400^{a)} | 0,25 % | 0,5 % |
| Copolymère séquencé cationique^{b)} | 0,25 % | - |
| Eau | q.s.p. 100 % | q.s.p. 100 % |

| | | |
|---|---|---|
| a) éther de cellulose à groupes ammonium quaternaire commercialisé par la société Amerchol, | | |
| b) copolymère séquencé cationique formé d'un bloc polystyrène et d'un bloc de poly(iodure de N-méthyl-4-vinylpyridinium) commercialisé par la société Polymer Source, Inc (dibloc polystyrène(18600g/mole)-poly(iodure de N-méthyl-4-vinylpyridinium)(131300 g/mole)). m.a. = matière active | | |

On lave des mèches de cheveux naturels avec chacun des shampooings ci-dessus et on les soumet, après séchage, à une évaluation par dix experts. Neuf experts sur dix trouvent que la mèche lavée avec le shampooing A, selon l'invention, a un toucher plus lisse et présente plus de corps que la mèche lavée avec le shampooing B selon l'état de la technique.

### Exemple 2 :

On a préparé le shampooing de composition suivante :

| | |
|---|---|
| | |
| Lauryléthersulfate de sodium (2OE) | 17 % m.a. |
| Cocobétaine | 2,5 % m.a. |
| JR 400 ^{a)} | 0,25 % |
| Copolymère séquencé anionique ^{b)} | 0,25 % |
| Eau | q.s.p. 100 % |

| | |
|---|---|
| b) polystyrène-b-acide polyacrylique, 2000 g/mole PS, 10500 g/mole PAA, commercialisé sous la référence P2476-SANa par la société "Polymer Source Inc." | |

La composition a les mêmes effets que ceux de la composition A de l'exemple 1.

## Revendications

1. Composition de lavage des matières kératiniques, de préférence des fibres kératiniques contenant, dans un milieu aqueux ou hydroalcoolique cosmétiquement acceptable,
• au moins un copolymère linéaire séquencé comprenant au moins un bloc hydrophobe et au moins un bloc hydrophile, à l'exclusion des copolymères séquencés d'oxyde d'éthylène et d'oxyde de propylène, des copolymères séquencés à motifs uréthanne et des copolymères séquencés à motifs siloxane,
• au moins un polymère, cationique ou amphotère, et
• au moins un agent tensioactif anionique associé à au moins un agent tensioactif non-ionique et/ou au moins un agent tensioactif amphotère.

2. Composition de lavage selon la revendication 1, **caractérisée par le fait que** le copolymère linéaire séquencé, éventuellement neutralisé, est dissous ou finement dispersé dans le milieu aqueux ou hydroalcoolique.

3. Composition de lavage selon la revendication 2, **caractérisée par le fait que** le copolymère linéaire séquencé, éventuellement neutralisé, est soluble dans l'eau.

4. Composition de lavage selon l'une quelconque des revendications précédentes, **caractérisée par le fait que** le ou les blocs hydrophiles du copolymère linéaire séquencé sont formés de monomères hydrosolubles choisis parmi les monomères hydrosolubles anioniques, les monomères hydrosolubles non-ioniques, les monomères hydrosolubles cationiques ou un mélange de ceux-ci.

5. Composition de lavage selon la revendication 4, **caractérisée par le fait que** les monomères hydrosolubles anioniques sont choisis parmi les acides carboxyliques à insaturation éthylénique, l'acide 2-acrylamido-2-méthylpropanesulfonique, l'acide styrènesulfonique, l'acide vinylsulfonique et l'acide vinylphosphonique.

6. Composition de lavage selon la revendication 4, **caractérisée par le fait que** les monomères hydrosolubles non-ioniques sont choisis parmi l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique, l'oxyde d'éthylène, l'acrylate d'hydroxyéthyle, l'acrylate d'hydroxypropyle, le méthacrylate d'hydroxyéthyle et le méthacrylate d'hydroxypropyle.

7. Composition de lavage selon la revendication 4, **caractérisée par le fait que** les monomères hydrosolubles cationiques sont choisis parmi le chlorure de diméthyldiallylammonium, le chlorure de méthylvinylimidazolium, la 2-vinylpyridine, la 4-vinylpyridine, la 2-méthyl-5-vinylpyridine, les halogénures de N-(alkyle en C₁₋₄)-4-vinylpyridinium, la vinylamine, les monomères de formule
H₂C=CR₁-CO-X₂
dans laquelle
R₁ représente un atome d'hydrogène ou un groupe méthyle,
X₂ représente un groupe hydrocarboné linéaire ou ramifié en C₁₋₆ portant au moins une fonction amine primaire, secondaire, tertiaire ou au moins un atome d'azote quaternaire, ou un groupe de formule NHR₂ ou de formule NR₂R₃ où R₂ et R₃ représentent indépendamment l'un de l'autre chacun un groupe hydrocarboné en C₁₋₆, linéaire ou ramifié, portant au moins une fonction amine primaire, secondaire, tertiaire ou au moins un atome d'azote quaternaire.

8. Composition de lavage selon l'une quelconque des revendications précédentes, **caractérisée par le fait que** le bloc hydrophobe est formé de monomères insolubles dans l'eau, choisis parmi les monomères vinylaromatiques, les diènes et les dérivés alkylés des diènes, le chloroprène, les acrylates d'alkyle en C₁₋₁₀, d'aryle en C₆₋₁₀ ou d'aralkyle en C₇₋₂₀, les méthacrylates d'alkyle en C₁₋₁₀, d'aryle en C₆₋₁₀ ou d'aralkyle en C₇₋₂₀, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène et les monomères vinyliques fluorés ou à chaîne perfluorée.

9. Composition de lavage selon l'une quelconque des revendications précédentes, **caractérisée par le fait que** le bloc hydrophile contient jusqu'à 25 % en moles, de préférence jusqu'à 10 % en moles, et idéalement jusqu'à 5 % en moles, d'un ou de plusieurs monomères insolubles dans l'eau selon la revendication 8.

10. Composition de lavage selon l'une quelconque des revendications précédentes, **caractérisée par le fait que** le bloc hydrophobe contient jusqu'à 25 % en moles, de préférence jusqu'à 10 % en moles, et idéalement jusqu'à 5 % en moles, d'un ou de plusieurs monomères hydrosolubles selon l'une quelconque des revendications 4 à 7.

11. Composition de lavage selon l'une quelconque des revendications précédentes, **caractérisée par le fait que** le copolymère séquencé est présent en une concentration allant de 0,01 à 20 %, de préférence de 0,1 à 5 %, rapportée au poids total de la composition de lavage.

12. Composition de lavage selon l'une quelconque des revendications précédentes, **caractérisée par le fait que** le polymère cationique ou amphotère est un polymère synthétique ou un polymère obtenu par modification chimique de polysaccharides.

13. Composition de lavage selon la revendication 12, **caractérisée par le fait que** le polymère cationique est choisi parmi les homopolymères ou copolymères d'esters ou d'amides acryliques ou méthacryliques à fonction aminée, les polymères à motifs pipérazinyle et à motifs alkylène ou hydroxyalkylène, les polyaminoamides solubles dans l'eau, les cyclopolymères d'alkyidiallylamine ou de dialkyldiallylammonium, les polymères de diammonium quaternaire, les polymères de polyammonium quaternaire, les polymères quaternaires de vinylpyrrolidone et de vinylimidazole, les polyamines, les polymères réticulés ou non-réticulés de sels de méthacryloyloxyalkyl(C₁₋₄)trialkyl(C₁₋₄)ammonium, les dérivés d'éther de cellulose comportant des groupements ammonium quaternaire, les dérivés cationiques de cellulose, les gommes de guar cationiques et les chitosanes cationiques.

14. Composition de lavage selon la revendication 12, **caractérisée par le fait que** le polymère amphotère est choisi parmi les copolymères de chlorure de diméthyldiallylammonium et d'acide acrylique, les terpolymères de chlorure de diméthyldiallylammonium, d'acrylamide et d'acide acrylique, les terpolymères de chlorure d'acrylamidopropyltriméthylammonium, d'acrylamide et d'acide 2-amidopropanesulfonique, les terpolymères de chlorure de méthacrylamidopropyltriméthylammonium, d'acrylate de méthyle et d'acide acrylique, les gommes de guar portant à la fois des groupes cationiques tels qu'amine primaire, secondaire ou tertiaire, ammonium, sulfonium ou phosphonium, et des groupes anioniques tels que carboxyle, sulfonate, sulfate, phosphaté ou phosphonate, les dérivés amphotères d'éthers de cellulose et des chitosanes à groupes carboxyle.

15. Composition de lavage selon l'une quelconque des revendications précédentes, **caractérisée par le fait que** les polymères cationiques ou amphotères sont des polymères filmogènes.

16. Composition de lavage selon l'une quelconque des revendications précédentes, **caractérisée par le fait que** le polymère cationique ou amphotère est présent en une concentration allant de 0,001 % à 20 % en poids, de préférence de 0,01 à 5 % en poids, rapportée au poids total de la composition de lavage.

17. Composition de lavage selon l'une quelconque des revendications précédentes, **caractérisée par le fait que** le ou les agents tensioactifs anioniques sont choisis parmi les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

18. Composition de lavage selon l'une quelconque des revendications précédentes, **caractérisée par le fait que** la concentration d'agents tensioactifs anioniques est comprise entre 3 et 35 % en poids, de préférence entre 5 et 25 % en poids, rapportée au poids total de la composition.

19. Composition de lavage selon l'une quelconque des revendications précédentes, **caractérisée par le fait que** l'agent tensioactif non-ionique est un (alkyle en C₆₋₂₄)-polyglycoside

20. Composition de lavage selon l'une quelconque des revendications précédentes, **caractérisée par le fait que** l'agent tensioactif amphotère est choisi parmi les (alkyle en C₈₋₂₀)-bétaïnes, les (alkyle en C₈₋₂₀)-amido(alkyle en C₆₋₈)bétaïnes, les alkylamphodiacétates et leurs mélanges.

21. Composition de lavage selon l'une quelconque des revendications précédentes, **caractérisée par le fait que** la quantité totale d'agents tensioactifs amphotères et/ou non ioniques est comprise entre 0,5 et 30 %, et en particulier entre 1 et 20 %, rapportée au poids total de la composition.

22. Utilisation d'une composition selon l'une quelconque des revendications précédentes pour le lavage des matières kératiniques, de préférence des fibres kératiniques.

## Patentansprüche

1. Zusammensetzung zur Reinigung von Keratinsubstanzen und vorzugsweise zur Reinigung von Keratinfasern, die in einem kosmetisch akzeptablen, wässerigen oder wässerig-alkoholischen Medium enthält:
- mindestens ein lineares Sequenzcopolymer, das mindestens einen hydrophoben Block und mindestens einen hydrophilen Block aufweist, wobei Sequenzcopolymere von Ethylenoxid und Propylenoxid, Sequenzcopolymere mit Urethaneinheiten und Sequenzcopolymere mit Siloxaneinheiten ausgenommen sind,
- mindestens ein kationisches oder amphoteres Polymer, und
- mindestens einen anionischen grenzflächenaktiven Stoff in Kombination mit mindestens einem nichtionischen grenzflächenaktiven Stoff und/ oder mindestens einem amphoteren grenzflächenaktiven Stoff.

2. Zusammensetzung zur Reinigung nach Anspruch 1, **dadurch gekennzeichnet, dass** das gegebenenfalls neutralisierte, lineare Sequenzcopolymer in dem wässerigen oder wässerig-alkoholischen Medium gelöst oder fein dispergiert ist.

3. Zusammensetzung zur Reinigung nach Anspruch 2, **dadurch gekennzeichnet, dass** das gegebenenfalls neutralisierte, lineare Sequenzcopolymer in Wasser löslich ist.

4. Zusammensetzung zur Reinigung nach einem der vorhergehenden Ansprüche, **dadurch gekennzeichnet, dass** der hydrophile Block oder die hydrophilen Blöcke des linearen Sequenzcopolymers aus wasserlöslichen Monomeren aufgebaut sind, die unter den anionischen wasserlöslichen Monomeren, nichtionischen wasserlöslichen Monomeren, kationischen wasserlöslichen Monomeren oder deren Gemischen ausgewählt sind.

5. Zusammensetzung zur Reinigung nach Anspruch 4, **dadurch gekennzeichnet, dass** die anionischen wasserlöslichen Monomere unter den ethylenisch ungesättigten Carbonsäuren, 2-Acrylamido-2-methylpropansulfonsäure, Styrolsulfonsäure, Vinylsulfonsäure und Vinylphosphonsäure ausgewählt sind.

6. Zusammensetzung zur Reinigung nach Anspruch 4, **dadurch gekennzeichnet, dass** die nichtionischen wasserlöslichen Monomere unter Acrylamid, N-C₁₋₆-alkylierten Acrylamiden oder N,N-C₁₋₃-dialkylierten Acrylamiden, Polyethylenglykolacrylat, Polyethylenglykolmethacrylat, N-Vinylacetamid, N-Methyl-N-vinylacetamid, N-Vinylformamid, N-Methyl-N-vinylformamid, N-Vinyllactamen, die eine cyclische Gruppe mit 4 bis 9 Kohlenstoffatomen enthalten, Vinylalkohol, Ethylenoxid, Hydroxyethylacrylat, Hydroxypropylacrylat, Hydroxyethylmethacrylat und Hydroxypropylmethacrylat ausgewählt sind.

7. Zusammensetzung zur Reinigung nach Anspruch 4, **dadurch gekennzeichnet, dass** die kationischen wasserlöslichen Monomere unter Dimethyldiallylammoniumchlorid, Methylvinylimidazoliumchlorid, 2-Vinylpyridin, 4-Vinylpyridin, 2-Methyl-5-vinylpyridin, N-(C₁₋₄-Alkyl)-4-vinylpyridiniumhalogeniden, Vinylamin und den Monomeren der Formel
H₂C=CR₁-CO-X₂
ausgewählt sind, worin bedeuten:
R₁ ein Wasserstoffatom oder die Methylgruppe,
X₂ eine geradkettige oder verzweigte Kohlenwasserstoffgruppe mit 1 bis 6 Kohlenstoffatomen, die mindestens eine primäre, sekundäre oder tertiäre Aminogruppe oder mindestens ein quartäres Stickstoffatom aufweist, oder eine Gruppe der Formel NHR₂ oder der Formel NR₂R₃, worin R₂ und R₃ unabhängig voneinander jeweils eine geradkettige oder verzweigte Kohlenwasserstoffgruppe mit 1 bis 6 Kohlenstoffatomen bedeuten, die mindestens eine primäre, sekundäre, tertiäre Aminofunktion oder mindestens ein quartäres Stickstoffatom aufweist.

8. Zusammensetzung zur Reinigung nach einem der vorhergehenden Ansprüche, **dadurch gekennzeichnet, dass** der hydrophobe Block von in Wasser unlöslichen Monomeren gebildet wird, die unter den vinylaromatischen Monomeren, Dienen, alkylierten Dienderivaten, Chloropren, C₁₋₁₀-Alkylacrylaten, C₆₋₁₀-Arylacrylaten oder C₇₋₂₀-Aralkylacrylaten, C₁₋₁₀-Alkylmethacrylaten, C₆₋₁₀-Arylmethacrylaten oder C₇₋₂₀-Aralkylmethacrylaten, Vinylacetat, Vinylethern der Formel CH₂=CH-O-R und Allylethern der Formel CH₂=CH-CH₂-O-R, worin R eine C₁₋₆-Alkylgruppe bedeutet, Acrylnitril, Vinylchlorid, Vinylidenchlorid, Caprolacton, Ethylen, Propylen und fluorierten Vinylmonomeren oder Vinylmonomeren mit perfluorierter Kette ausgewählt sind.

9. Zusammensetzung zur Reinigung nach einem der vorhergehenden Ansprüche, **dadurch gekennzeichnet, dass** der hydrophile Block bis zu 25 Mol-%, vorzugsweise bis zu 10 Mol-% und idealerweise bis zu 5 Mol-% eines oder mehrerer in Wasser unlöslicher Monomere nach Anspruch 8 enthält.

10. Zusammensetzung zur Reinigung nach einem der vorhergehenden Ansprüche, **dadurch gekennzeichnet, dass** der hydrophobe Block bis zu 25 Mol-%, vorzugsweise bis zu 10 Mol-% und idealerweise bis zu 5 Mol-% eines oder mehrerer wasserlöslicher Monomere nach einem der Ansprüche 4 bis 7 enthält.

11. Zusammensetzung zur Reinigung nach einem der vorhergehenden Ansprüche, **dadurch gekennzeichnet, dass** das Sequenzcopolymer in einer Konzentration von 0,01 bis 20 % und vorzugsweise 0,1 bis 5 %, bezogen auf das Gesamtgewicht der Reinigungszusammensetzung, enthalten ist.

12. Zusammensetzung zur Reinigung nach einem der vorhergehenden Ansprüche, **dadurch gekennzeichnet, dass** das kationische oder amphotere Polymer ein synthetisches Polymer oder ein durch chemische Modifikation von Polysacchariden erhaltenes Polymer ist.

13. Zusammensetzung zur Reinigung nach Anspruch 12, **dadurch gekennzeichnet, dass** das kationische Polymer unter den Homopolymeren oder Copolymeren von Acrylestern oder Acrylamiden oder Methacrylestern oder Methacrylamiden mit Aminofunktion, Polymeren mit Piperazinyleinheiten und Alkylen- oder Hydroxyalkyleneinheiten, in Wasser löslichen Polyaminoamiden, Alkyldiallylamin-Cyclopolymeren oder Dialkyldiallylammonium-Cyclopolmeren, quartären Diammoniumpolymeren, quartären Polyammoniumpolymeren, quartären Polymeren von Vinylpyrrolidon und Vinylimidazol, Polyaminen, vernetzten oder nicht vernetzten Polymeren von Methacryloyloxyalkyl(C₁₋₄)trialkyl(C₁₋₄)-ammoniumsalzen, Celluloseetherderivaten, die quartäre Ammoniumgruppen enthalten, kationischen Cellulosederivaten, kationischen Guargummen und kationischen Chitosanen ausgewählt ist.

14. Zusammensetzung zur Reinigung nach Anspruch 12, **dadurch gekennzeichnet, dass** das amphotere Polymer unter den Copolymeren von Dimethyldiallylammoniumchlorid und Acrylsäure, Terpolymeren von Dimethyldiallylammoniumchlorid, Acrylamid und Acrylsäure, Terpolymeren von Acrylamidopropyltrimethylammoniumchlorid, Acrylamid und 2-Amidopropansulfonsäure, Terpolymeren von Methacrylamidopropyltrimethylammoniumchlorid, Methylacrylat und Acrylsäure, Guargummen, die gleichzeitig kationische Gruppen, wie primäre, sekundäre oder tertiäre Aminogruppen, Ammoniumgruppen, Sulfoniumgruppen oder Phosphoniumgruppen, und anionische Gruppen enthalten, beispielsweise Carboxy, Sulfonat, Sulfat, Phosphat oder Phosphonat, amphoteren Celluloseetherderivaten und Chitosanen mit Carboxygruppen ausgewählt ist.

15. Zusammensetzung zur Reinigung nach einem der vorhergehenden Ansprüche, **dadurch gekennzeichnet, dass** die kationischen oder amphoteren Polymere filmbildende Polymere sind.

16. Zusammensetzung zur Reinigung nach einem der vorhergehenden Ansprüche, **dadurch gekennzeichnet, dass** das kationische oder amphotere Polymer in einer Konzentration von 0,001 bis 20 Gew.-% und vorzugsweise 0,01 bis 5 Gew.-%, bezogen auf das Gesamtgewicht der Reinigungszusammensetzung, enthalten ist.

17. Zusammensetzung zur Reinigung nach einem der vorhergehenden Ansprüche, **dadurch gekennzeichnet, dass** der anionische grenzflächenaktive Stoff oder die anionischen grenzflächenaktiven Stoffe unter den Alkylsulfaten, Alkylethersulfaten und Alkylethercarboxylaten und deren Gemischen insbesondere in Form der Alkali- oder Erdalkalisalze, Ammoniumsalze, Aminsalze oder Aminoalkoholsalze ausgewählt sind.

18. Zusammensetzung zur Reinigung nach einem der vorhergehenden Ansprüche, **dadurch gekennzeichnet, dass** die Konzentration der anionischen grenzflächenaktiven Stoffe im Bereich von 3 bis 35 Gew.-% und vorzugsweise 5 bis 25 Gew.-%, bezogen auf das Gesamtgewicht der Zusammensetzung, liegt.

19. Zusammensetzung zur Reinigung nach einem der vorhergehenden Ansprüche, **dadurch gekennzeichnet, dass** der nicht ionische grenzflächenaktive Stoff ein C₆₋₂₄-Alkylpolyglycosid ist.

20. Zusammensetzung zur Reinigung nach einem der vorhergehenden Ansprüche, **dadurch gekennzeichnet, dass** der amphotere grenzflächenaktive Stoff unter den C₈₋₂₀-Betainen, C₈₋₂₀-Alkylamido(C₆₋₈-alkyl)betainen, Alkylamphodiacetaten und deren Gemischen ausgewählt ist.

21. Zusammensetzung zur Reinigung nach einem der vorhergehenden Ansprüche, **dadurch gekennzeichnet, dass** das Gesamtgewicht der amphoteren und/oder nicht ionischen grenzflächenaktiven Stoffe im Bereich von 0,5 bis 30 % und insbesondere 1 bis 20 %, bezogen auf das Gesamtgewicht der Zusammensetzung, liegt.

22. Verwendung einer Zusammensetzung nach einem der vorhergehenden Ansprüche zur Reinigung von Keratinmaterialien und vorzugsweise Keratinfasern.

## Claims

1. Composition for washing keratinous substances, preferably keratinous fibres, comprising, in a cosmetically acceptable aqueous or hydroalcoholic medium,
• at least one linear block copolymer comprising at least one hydrophobic block and at least one hydrophilic block, with the exception of block copolymers of ethylene oxide and of propylene oxide, block copolymers comprising urethane units and block copolymers comprising siloxane units,
• at least one cationic or amphoteric polymer, and
• at least one anionic surface-active agent in combination with at least one nonionic surface-active agent and/or at least one amphoteric surface-active agent.

2. Washing composition according to Claim 1, **characterized in that** the linear block copolymer, optionally neutralized, is dissolved or finely dispersed in the aqueous or hydroalcoholic medium.

3. Washing composition according to Claim 2, **characterized in that** the linear block copolymer, optionally neutralized, is soluble in water.

4. Washing composition according to any one of the preceding claims, **characterized in that** the hydrophilic block or blocks of the linear block copolymer are formed of water-soluble monomers chosen from anionic water-soluble monomers, nonionic water-soluble monomers, cationic water-soluble monomers or a mixture of these.

5. Washing composition according to Claim 4, **characterized in that** the anionic water-soluble monomers are chosen from carboxylic acids comprising ethylenic unsaturation, 2-acrylamido-2-methylpropanesulphonic acid, styrenesulphonic acid, vinylsulphonic acid and vinylphosphonic acid.

6. Washing composition according to Claim 4,
**characterized in that** the nonionic water-soluble monomers are chosen from acrylamide, N-(C₁₋₆ alkylated)acrylamides or N,N-di(C₁₋₃ alkylated)acrylamides, polyethylene glycol acrylate, polyethylene glycol methacrylate, N-vinylacetamide, N-methyl-N-vinylacetamide, N-vinylformamide, N-methyl-N-vinylformamide, N-vinyllactams comprising a cyclic group of 4 to 9 carbon atoms, vinyl alcohol, ethylene oxide, hydroxyethyl acrylate, hydroxypropyl acrylate, hydroxyethyl methacrylate and hydroxypropyl methacrylate.

7. Washing composition according to Claim 4, **characterized in that** the cationic water-soluble monomers are chosen from dimethyldiallylammonium chloride, methylvinylimidazolium chloride, 2-vinylpyridine, 4-vinylpyridine, 2-methyl-5-vinylpyridine, N-(C₁₋₄ alkyl)-4-vinylpyridinium halides, vinylamine or monomers of formula
H₂C=CR₁-CO-X₂
in which
R₁ represents a hydrogen atom or a methyl group,
X₂ represents a linear or branched C₁₋₆ hydrocarbonaceous group carrying at least one primary, secondary or tertiary amine functional group or at least one quaternary nitrogen atom, or a group of formula NHR₂ or of formula NR₂R₃ where R₂ and R₃ each represent, independently of one another, a linear or branched C₁₋₆ hydrocarbonaceous group carrying at least one primary, secondary or tertiary amine functional group or at least one quaternary nitrogen atom.

8. Washing composition according to any one of the preceding claims, **characterized in that** the hydrophobic block is formed from water-insoluble monomers chosen from vinylaromatic monomers, dienes and alkylated derivatives of dienes, chloroprene, C₁₋₁₀ alkyl, C₆₋₁₀ aryl or C₇₋₂₀ aralkyl acrylates, C₁₋₁₀ alkyl, C₆₋₁₀ aryl or C₇₋₂₀ aralkyl methacrylates, vinyl acetate, vinyl ethers of formula CH₂=CH-O-R and allyl ethers of formula CH₂=CH-CH₂-O-R where R represents a C₁₋₆ alkyl group, acrylonitrile, vinyl chloride, vinylidene chloride, caprolactone, ethylene, propylene, and fluorinated vinyl monomers or vinyl monomers comprising a perfluorinated chain.

9. Washing composition according to any one of the preceding claims, **characterized in that** the hydrophilic block comprises up to 25 mol%, preferably up to 10 mol% and ideally up to 5 mol% of one or more water-insoluble monomers according to Claim 8.

10. Washing composition according to any one of the preceding claims, **characterized in that** the hydrophobic block comprises up to 25 mol%, preferably up to 10 mol% and ideally up to 5 mol% of one or more water-soluble monomers according to any one of Claims 4 to 7.

11. Washing composition according to any one of the preceding claims, **characterized in that** the block copolymer is present in a concentration ranging from 0.01 to 20%, preferably from 0.1 to 5%, with respect to the total weight of the washing composition.

12. Washing composition according to any one of the preceding claims, **characterized in that** the cationic or amphoteric polymer is a synthetic polymer or a polymer obtained by chemical modification of polysaccharides.

13. Washing composition according to Claim 12, **characterized in that** the cationic polymer is chosen from homopolymers or copolymers of acrylic esters, methacrylic esters, acrylamides or methacrylamides comprising an amine functional group, polymers comprising piperazinyl units and comprising alkylene or hydroxyalkylene units, water-soluble polyaminoamides, cyclopolymers of alkyldiallylamine or of dialkyldiallylammonium, diquaternary ammonium polymers, polyquaternary ammonium polymers, quaternary polymers of vinylpyrrolidone and of vinylimidazole, polyamines, crosslinked or noncrosslinked polymers of methacryloyloxy (C₁₋₄)alkyltri (C₁₋₄)alkylammonium salts, cellulose ether derivatives comprising quaternary ammonium groups, cationic cellulose derivatives, cationic guar gums and cationic chitosans.

14. Washing composition according to Claim 12, **characterized in that** the amphoteric polymer is chosen from copolymers of dimethyldiallylammonium chloride and of acrylic acid, terpolymers of dimethyldiallylammonium chloride, of acrylamide and of acrylic acid, terpolymers of acrylamidopropyltrimethylammonium chloride, of acrylamide and of 2-amidopropanesulphonic acid, terpolymers of methacrylamidopropyltrimethylammonium chloride, of methyl acrylate and of acrylic acid, guar gums carrying both cationic groups, such as primary, secondary or tertiary amine groups or ammonium, sulphonium or phosphonium groups, and anionic groups, such as carboxyl, sulphonate, sulphate, phosphate or phosphonate groups, amphoteric derivatives of cellulose ethers and chitosans comprising carboxyl groups.

15. Washing composition according to any one of the preceding claims, **characterized in that** the cationic or amphoteric polymers are film-forming polymers.

16. Washing composition according to any one of the preceding claims, **characterized in that** the cationic or amphoteric polymer is present in a concentration ranging from 0.001% to 20% by weight, preferably from 0.01 to 5% by weight, with respect to the total weight of the washing composition.

17. Washing composition according to any one of the preceding claims, **characterized in that** the anionic surface-active agent or agents are chosen from alkyl sulphates, alkyl ether sulphates and alkyl ether carboxylates, and their mixtures, in particular in the form of alkali metal or alkaline earth metal, ammonium, amine or aminoalcohol salts.

18. Washing composition according to any one of the preceding claims, **characterized in that** the concentration of anionic surface-active agents is between 3 and 35% by weight, preferably between 5 and 25% by weight, with respect to the total weight of the composition.

19. Washing composition according to any one of the preceding claims, **characterized in that** the nonionic surface-active agent is a (C₆₋₂₄ alkyl)polyglycoside.

20. Washing composition according to any one of the preceding claims, **characterized in that** the amphoteric surface-active agent is chosen from (C₈₋₂₀ alkyl) betaines, (C₈₋₂₀ alkyl) amido(C₆₋₈ alkyl) betaines, alkylamphodiacetates and their mixtures.

21. Washing composition according to any one of the preceding claims, **characterized in that** the total amount of amphoteric and/or nonionic surface-active agents is between 0.5 and 30% and in particular between 1 and 20% with respect to the total weight of the composition.

22. Use of a composition according to any one of the preceding claims for washing keratinous substances, preferably keratinous fibres.
